(19) Europäisches Patentamt
European Patent Office
Office européen des brevets

(11) Publication number: **0 081 621 A1**

# (12) EUROPEAN PATENT APPLICATION

(21) Application number: 82101592.2

(22) Date of filing: 02.03.82

(51) Int. Cl.³: C 07 D 311/30, C 07 D 311/22 // A61K31/35

(30) Priority: 14.12.81 US 330122

(43) Date of publication of application: 22.06.83 Bulletin 83/25

(84) Designated Contracting States: AT BE CH DE FR GB IT LI LU NL SE

(71) Applicant: PENNWALT CORPORATION, Pennwalt Building Three Parkway, Philadelphia Pennsylvania 19102 (US)

(72) Inventor: Wu, Edwin Shen-Chou, 147 Ashley Drive, Rochester New York 14620 (US)

(74) Representative: Kraus, Walter, Dr. et al, Patentanwälte Dres. Kraus & Weisert Irmgardstrasse 15, D-8000 München 71 (DE)

## (54) Chromones.

(57) Chromone derivatives such as 7-(3-n-propylamino-2-hydroxypropoxy)-2,3-diphenylchromone which are useful as antihypertensive agents.

EP 0 081 621 A1

# CHROMONES

## Background of the Invention

This is a continuation-in-part of my copending applications, U. S. Serial No. 259,387 and U. S. Serial No. 259,403.

This invention relates to chromone derivatives substituted by 3-amino-2-hydroxypropoxy side chains, medicinal preparations containing the same, and the utilization of such preparations as antihypertensive agents in animals.

Compounds related to propranolol, a potent beta-blocker, constitute a large class of compounds which have profound effects on the cardiovascular system and have found utility as antihypertensive, antidysrhythmic and antianginal drugs. However, the beta-blocking properties of these compounds are often undesirable, especially in patients with coronary insufficiencies and bronchial diseases. A compound devoid of beta-blocking effect but retaining antihypertensive effect on the blood pressure of warm-blooded animals has therefore long been sought.

Several patents disclose various compounds which either have beta-blocking properties or do not have antihypertensive effects. U. S. Patent No. 3,891,651 discloses compounds which are amides and, it is thought, the nitrogen of the amide which is contained in the isoquinoline fragment is likely to be responsible for any activity in that compound. U. S. Patent No. 3,816,470 discloses various salts of secondary amines with chromone-2-carboxylic acids. U. S. Patent No. 3,812,156 discloses a method of preparing ethyl flavone-7-oxyacetate. U. S. Patent No. 3,352,754 discloses simple 7-hydroxy or 7-alkoxy isoflavones which are not amines and which are used for various inflammatory disorders. U. S. Patent No. 3,219,531 discloses 5,7-dioxyacetic acid flavone compounds, but no

amine functions are present. U. S. Patent No. 3,046,275 discloses 7-dialkylaminoalkoxy derivatives but does not contain any of the hydroxyl groups of the side chain which is central for activity. Various monodialkylaminoethyl ethers of quercetin are disclosed in U. S. Patent No. 2,861,992, but do not contain 3-amino-2-hydroxypropoxy side chains. Also not containing that side chain are the compounds disclosed in U. S. Patent No. 2,897,211.

Several flavones are disclosed in an article published in Acta Pharmaceutica Sinica,15 253 (1980) in the People's Republic of China, where lack of beta-blocking is reported. There is no suggestion that the compounds have antihypertensive properties.

## Summary of the Invention

It has now been found that a marked reduction in the blood pressure of warm-blooded animals can be achieved, without beta-blocking effects in the beta-adrenergic nervous system, by administering to the animal, in an amount effective to reduce hypertension, a chromone of the formula:

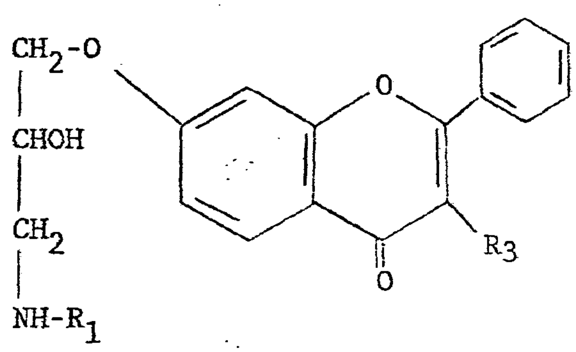

or a pharmaceutically acceptable salt thereof wherein $R_1$ is i-propyl, n-propyl, or cyclopropyl, $R_3$ is phenyl or hydrogen and provided, however, that $R_1$ is cyclopropyl or n-propyl when $R_3$ is hydrogen.

These compounds have unexpectedly superior antihypertensive properties, especially in terms of being

able to achieve long-term activity (e.g., 24 hours) at relatively low dosages.

The compounds are usually mixed with a pharmaceutical carrier so that the composition for commercial use contains 0.5 to 20% by weight of the compound.

The compositions are normally adapted for peroral or parenteral use, but may be used in other forms such as suppositories. The peroral compositions are preferably in the form of tablets, capsules or suspensions, while the parenteral composition is preferably an injectable solution or suspension.

Examples of suitable inert pharmaceutical carriers are celluloses (particularly microcrystalline celluloses), sugar syrups, potato starch, talcum, polyethylene glycols and lactose.

Examples of suitable acids for forming the acid addition salts are maleic acid, hydrochloric acid, sulfuric acid, phosphoric acid, methanesulfonic acid, tartaric acid, citric acid, and the cation exchange resins such as the carboxylic acid, phosphonic acid and sulfonic acid resins.

For sustained release, a coated complex of the compound absorbed onto an ion exchange resin may be employed in accordance with the teaching of British Patent 1,544,761.

The usual peroral dosage of the compound is 0.1 to 150 mg. per day (preferably 0.1 to 50 mg.) while the parenteral dosage is normally 0.1 to 40 mg. per day (preferably 0.1 to 10 mg.)

The capsules, tablets, syrups and suspensions of the compounds are prepared by conventional procedures.

It should be noted that the compounds of this invention are antihypertensive agents, not hypotensive agents, i.e., they reduce the blood pressure to normal but not below normal.

Detailed Description

The compounds of formula (1) above can be prepared by reacting epichlorohydrin or epibromohydrin with a compounds of the formula

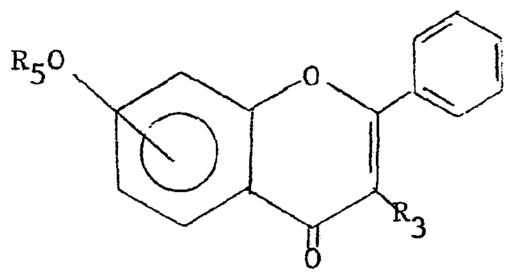

(2),

wherein $R_3$ is the same as defined above and $OR_5$ represents a hydroxyl group substituted on the 7 position, in the presence of a solvent and a base to give a product of formula (2) where $OR_5$ is

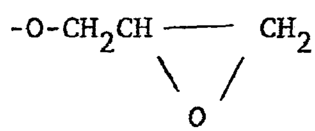

This epoxide is allowed to react with an amine, $R_1NH_2$ where $R_1$ is as above defined, in an alcoholic solvent at elevated temperature to afford a product of formula (1). P. Da Re et al., J. Med. Chem., Vol. 15, 868-869 (1972), describe the testing of chromones as in formula (1), but where $R_3$ is methyl, for beta-adrenergic blocking activity. Da Re et al. found that all the compounds were devoid of beta-blocking activity, suggesting that chromones would not be expected to have antihypertensive properties. Additionally, ethanolamine anologues of the Da Re et al. materials are disclosed in Vol. 15 pages 198-199 of the J. Med. Chem., (1972). These analogues are beta-blockers, typical of the pronethalol type which owe their activity to the 2-isopropylaminoethanol side chain. Typical 3-amino-2-hydroxypropoxy side chain furochromone compounds are disclosed in papers presented in Drugs of the Future, Vol. III, No. 8 (1978), pages 569-571; Drugs of the Future, Vol. III, No. 11, (1978),

pages 816-818; and <u>Therapie</u>, (1977), Vol. 32, pages 111-120. None of these references, of course, even suggest that antihypertensive activity may be possible with or without beta-blockade. None of these references disclose the compounds of this invention.

The following general procedures are used in the examples to follow:

A. <u>For Epoxides</u> - Epichlorohydrin or epibromohydrin (greater than 2 equivalents) is added in the presence or absence of nitrogen to a stirred solution or suspension of the hydroxy chromone derivative in solvents, such acetone, aqueous ethanol, 50% aqueous dimethylsulfoxide (DMSO) or water, containing a suitable base, such as potassium carbonate or sodium hydroxide, with or without sodium iodide. The reaction is allowed to proceed either at room temperature or elevated temperature and monitored by thin layer chromatography (tlc). Product formed as solid is then collected and washed with water. The mother liquor is diluted with water and extracted with chloroform ($CHCl_3$) to give more product. Where the product is soluble in solvents, the reaction mixture is filtered off and the solids washed with the solvent. The filtrate is evaporated to give a solid which is washed with water to get rid of remaining traces of the base. Yield is in the 60 to 80% range. This material is usually used directly for further reaction without purification.

B. <u>For Epoxide Ring-Opening With Amine and the Amine Salt Formation</u> - A white suspension of the chromone epoxide, amine, such as low (large excess) and high (10% excess) boiling point amines, an alcoholic solvent, methanol (A.R.) ethanol (abs.) or isopropyl alcohol, is heated at elevated temperatures until the starting material is gone (as followed by tlc). Since the reaction product is usually soluble in the alcohol, the

precipitate is filtered off and the filtrate is evaporated to give a viscous liquid which, upon addition of anhydrous ether or alcohol, crystallizes out. When the product is insoluble in alcohol at the end of the reaction, it is collected. Purification of the amine derivative is performed via acid-base work-up, column chromatography or recrystallization. The amine obtained is either suspended or dissolved in an alcohol, and then acidified with an alcohol solution saturated with hydrogen chloride or other acid to a pH of 1. The salt formed is precipitated out directly or by addition of anhydrous ether. The salt is then recrystallized from suitable solvents.

Example 1 - Preparation of 7-(2-hydroxy-3-isopropylaminopropoxy)-2,3-diphenylchromone hydrochloride:

7-(2,3-Epoxypropoxy)-2,3,-diphenylchromone - To a solution of 50% aqueous DMSO (280 ml), containing sodium hydroxide (5.60 g, 140 mmol) was added 7-hydroxy 2,3,-diphenylchromone (44.01 g, 140 mmol) and the resultant mixture was sitrred until most of the solid went into the solution. Epichlorohydrin was then introduced into the suspension. The solution was stirred at room temperature (RT) for 2.5 days and solid formed during the reaction was collected from time to time (about twice a day). The white solid (total = 47.58 g) was recrystallized from isopropyl alcohol several times to afford 28.15 g of relatively pure white prisms, m.p. 143-145°; yield 54%. The mother liquor was concentrated to produce more crystals.

7-(2-Hydroxy-3-isopropylaminopropoxy)-2,3-diphenylchromone hydrochloride - A white suspension of relatively pure 7-(2,3-epoxypropoxy)-2,3-diphenylchromone (27.78 g, 75 mmol), isopropylamine (18.8 ml), and methanol (A. R., 150 ml) was heated under nitrogen at 55°C until complete disappearance of the starting epoxide. The white precipitate (1.09 g) in the cooled reaction mixture was

filtered off, and the filtrate was evaporated to a golden syrup. Anhydrous ether was added, and white crystals (28.07 g) were obtained. It was column-chromatographed over silica gel. This purificaiton yielded 24.05 g of white crystals, m.p. 152-154° (75%).

The amine thus obtained was dissolved in 100 ml of MeOH A. R. and treated with saturated solution of hydrogen chloride in ethanol until the solution was pH = 1. The hydrochloride salt was precipitated out by addition of anhydrous ether. A white solid was collected and recrystallized from MeOH-ether (also decolorized with a small amount of activated charcoal) to produce 21.6 g (83% yield) of pure white crystals, m.p. 164-166°.

Anal. Calc'd. for $C_{27}H_{28}ClNO_4.H_2O$: C, 67.00; H, 6.25; Cl, 7.32; N, 2.89.

Found: C, 67.10; H, 5.95; Cl, 7.19; N, 2.65.

Example 2 - Preparation of 7-(3-*n*-propylamino-2-hydroxypropoxy)-2,3-diphenylchromone hydrochloride

The free base was prepared according to Method B from *n*-propylamine and 7-(2,3-epoxypropoxy)-2,3-diphenylchromone in 65% yield as white prisms, m.p. 140-142° (*i*PrOH).

The hydrochloride salt was obtained in 95% yield as white prisms, m.p. 134-136° (*i*PrOH)

Example 3 - Preparation of 7-(3-cyclopropylamino-2-hydroxypropoxy)flavone hydrochloride

Using Method B, the amine was prepared from cyclopropylamine and 7-(2,3-epoxypropoxy)flavone and purified by column chromatography, yield 26%. Treatment of the free base with HCl/EtOH solution gave the hydrochloride salt in 95% yield, m.p. 214° (MeOH-ether).

Anal. Calc'd. for $C_{21}H_{22}ClNO_4$: C, 65.03; H, 5.71; Cl, 9.14; N, 3.61; O, 16.49.

Found: C, 64.82; H, 5.81; Cl, 9.27; N, 3.65; O, 16.78.

Example 4 - Preparation of 7-(3-cyclopropylamino-2-hydroxypropoxy)-2,3-diphenylchromone

The amine was prepared according to Method B from cyclopropylamine and 7-(2,3-epoxypropoxy)-2,3-diphenylchromone and purified by high pressure liquid chromatography to give white crystals, m.p. 135-137° (MeOH-ether), in 25% yield.

Example 5 - Preparation of 7-(3-n-propylamino-2-hydroxypropoxy) flavone hydrochloride

Using Method B, the reaction of 7-(2,3-epoxypropoxy) -flavone and n-propylamine afforded the hydrochloride in 48% yield, m.p. 234-235° (methanol). Its corresponding free base had a m.p. of 140-145°.

Anal. Calc'd. for $C_{21}H_{24}ClNO_4$: C, 64.69; H, 6.20; Cl, 9.09; N, 3.59; H, 16.41.

Found: C, 64.60; H, 6.37; Cl, 8.44; N, 3.48; O, 16.57.

Example 6 - Preparation of 3-Methyl-7-(3-isopropylamino-2-hydroxypropoxy)flavone hydrochloride

3-Methyl-7-(2,3-epoxypropoxy)flavone - Using Method A, 3-methyl-7-hydroxyflavone was converted to its corresponding 7-epoxypropyl ether, m.p. 231-235° (crude); yield 86%.

3-Methyl-7-(2-hydroxy-3-isopropylaminopropoxy) flavone hydrochloride - The amine salt was prepared as described in Method B to give a white solid, m.p. 120-122° (EtOH-ether); yield 47%. The free base had a m.p. of 141-144°.

Anal. Calc'd. for $C_{22}H_{26}ClNO_4$: C, 65.42; H, 6.48; Cl, 8.77; N, 3.46; O, 15.84.

Found: C, 65.62; H, 6.61; Cl, 8.51; N, 3.33, O, 15.30.

This compound is disclosed in the P. Da Re article referred to herein above.

WHAT IS CLAIMED IS:

1. Chromone having the formula

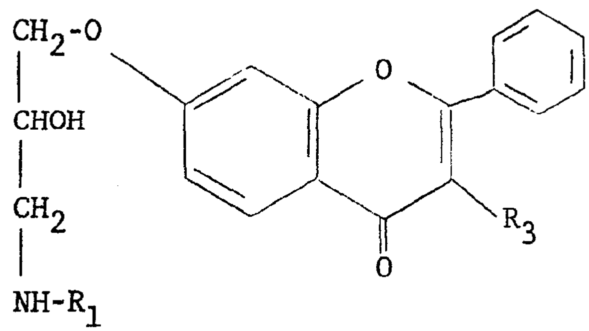

or a pharmaceutically acceptable salt thereof wherein $R_1$ is i-propyl, n-propyl, or cyclopropyl, $R_3$ is phenyl or hydrogen and provided, however, that $R_1$ is cyclopropyl or n-propyl when $R_3$ is hydrogen.

2. The chromone of claim 1 wherein $R_1$ is n-propyl.

3. The chromone of claim 1 wherein $R_3$ is phenyl.

4. The chromone of claim 3, 7-(3-n-propylamino-2-hydroxypropoxy)-2,3-diphenylchromone hydrochloride.

5. The chromone of claim 3, 7-(2-hydroxy-3-isopropylaminopropoxy)-2,3-diphenylchromone hydrochloride.

6. The chromone of claim 1, 7-(3-cyclopropylamino-2-hydroxypropoxy)flavone hydrochloride.

7. The chromone of claim 1, 7-(3-cyclopropylamino-2-hydroxypropoxy)-2,3-diphenylchromone.

8. The chromone of claim 1, 7-(3-n-propylamino-2-hydroxypropoxy)flavone hydrochloride.

CLAIMS FOR AUSTRIA:

1. Process for the production of a chromone having the formula (1)

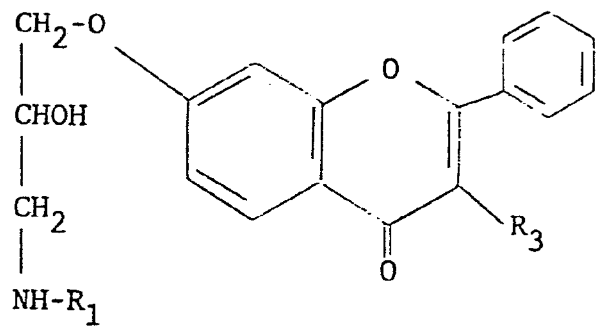

(1)

or a pharmaceutically acceptable salt thereof wherein $R_1$ is i-propyl, n-propyl, or cyclopropyl, $R_3$ is phenyl or hydrogen and provided, however, that $R_1$ is cyclopropyl or n-propyl when $R_3$ is hydrogen, characterized by reacting epichlorohydrin or epibromohydrin with a compound of the formula (2)

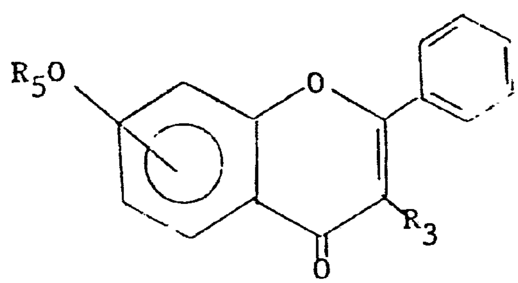

(2)

wherein $R_3$ is the same as defined above and $OR_5$ represents a hydroxyl group substituted on the 7 position, in the presence of a solvent and a base to give an epoxide product of formula (2) where $OR_5$ is

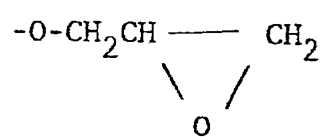

and reacting this epoxide with an amine, $R_1NH_2$ where $R_1$ is as above defined, in an alcoholic solvent at elevated temperature to afford a product of formula (1) and optional-

Example 7 - Preparation of 6-(2-Hydroxy-3-isopropylamino-propoxy) flavone hydrochloride:

6-(2,3-Epoxypropoxy) flavone - This epoxypropyl ether was obtained in 65% yield from 6-hydroxyflavone according to Method A.

6-(2-Hydroxy-3-isopropylaminopropoxy) flavone hydrochloride - The reaction of the above epoxide with isopropylamine according to Method B yielded (after recrystallization from n-butanol) 49% product, melting at 209-211°.

Anal. Calc'd. for $C_{21}H_{24}ClNO_4$: C, 64.69; H, 6.20; Cl, 9.09; N, 3.59; O, 16.41.

Found: C, 64.59; H, 6.61; Cl, 9.64; N, 3.40; O, 16.02.

This compound is disclosed in the People's Republic of China article published in Acta Pharmaceutica, 15 253 (1980), previously identified herein.

Example 8 - Pharmacology Data

Antihypertensive activity was determined in the spontaneously hypertensive rat. Animals were dosed with compound or control vehicle following a control blood pressure (systolic) and heart rate determination made by means of an inflatable tail cuff. Measurements were contained at 2, 5, 7.5 and 24 hours post dosing. Five animals per test group were employed. A 10% decrease in systolic blood pressure which was statistically different from both the control animals and the post dosage measurement is classified as minimal activity.

The following table gives minimal dosage required for minimal activity at 5 and 24 hours for the compounds of the prior examples.

Pharmacology Data

| Example No. | $R_1$ | $R_3$ | Minimum Effective Dosage mg./kg. 5 hours | 24 hours |
|---|---|---|---|---|
| 1 | i-propyl | phenyl | 8 | 17 |
| 2 | n-propyl | phenyl | 8 | 8 |
| 3 | cyclopropyl | hydrogen | 8 | 35 |
| 4 | cyclopropyl | phenyl | 8 | 24 |
| 5 | n-propyl | hydrogen | 10 | 45 |
| 6 | i-propyl | methyl | 75 | not effective |
| 7 | i-propyl | hydrogen | 75 | not effective |

These compounds do not exhibit beta-blocking activity when determined as antagonism of isoproterenol-induced beta stimulation of isolated rat heart or inhibition of isoproternol-induced effects on the cardiovascular system of the anesthetized rat.

ly converting the amine in its pharmaceutically acceptable salt.

2. The process of claim 1 wherein $R_1$ is n-propyl.

3. The process of claim 1 wherein $R_3$ is phenyl.

4. The process of claim 3 wherein 7-(3-n-propylamino-2-hydroxypropoxy)-2,3-diphenylchromone hydrochloride is prepared.

5. The process of claim 3 wherein 7-(2-hydroxy-3-isopropylaminopropoxy)-2,3-diphenylchromone hydrochloride is prepared.

6. The process of claim 1 wherein 7-(3-cyclopropylamino-2-hydroxypropoxy)flavone hydrochloride is prepared.

7. The process of claim 1 wherein 7-(3-cylcoproylamino-2-hydroxypropoxy)-2,3-diphenylchromone is prepared.

8. The process of claim 1 wherein 7-(3-n-propylamino-2-hydroxypropoxy)flavone hydrochloride is prepared.

European Patent Office

# EUROPEAN SEARCH REPORT

0081621

Application number

EP 82101592.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | EP - A1 - 0 031 885 (MERCK)<br>* Formula I *<br>-- | 1 | C 07 D 311/30<br>C 07 D 311/22//<br>A 61 K 31/35 |
| A | DE - A1 - 2 740 950 (MERCK)<br>* Formulas II,III *<br>-- | 1 | |
| D,A | US - A - 3 891 651 (BLAISE)<br>* Formula I; abstract *<br>-- | 1 | |
| D,A | US - A - 3 812 156 (BONOLA)<br>* Abstract *<br>-- | 1 | |
| D,A | US - A - 3 046 275 (KOHLSTAEDT)<br>* Claim 1 *<br>---- | 1 | TECHNICAL FIELDS SEARCHED (Int. Cl. 3)<br>C 07 D 311/00 |

The present search report has been drawn up for all claims

| Place of search | Date of completion of the search | Examiner |
|---|---|---|
| VIENNA | 11-02-1983 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82